# EUROPEAN PATENT APPLICATION

(11) **EP 4 636 791 A1**
(43) Date of publication of application: **22.10.2025**
(21) Application number: 23903429.1
(22) Date of filing: 08.12.2023
(51) Int. Cl.: H01F 6/02, A61N 5/10, H01F 6/00, H01F 6/04, H01F 6/06, H05H 7/04

(54) **SUPERCONDUCTING MAGNET DEVICE, PARTICLE BEAM THERAPY SYSTEM, AND METHOD OF CONTROLLING SUPERCONDUCTING MAGNET DEVICE**

(30) Priority: 13.12.2022 JP 2022198520
(71) Applicant: B dot Medical Inc., Tokyo 134-0003 (JP)
(72) Inventor: TAKESHITA, Eri, Tokyo 134-0003 (JP); KANAI, Yoshiharu, Tokyo 134-0003 (JP); WACHI, Yoshihiro, Tokyo 134-0003 (JP)
(74) Representative: J A Kemp LLP
(86) International application number: PCT/JP2023/043929
(87) International publication number: WO 2024/128142

(57) **Abstract**

A superconducting magnet device includes a first superconducting coil, a second superconducting coil, a first power source that supplies a current to the first superconducting coil and does not supply a current to the second superconducting coil, a second power source that does not supply a current to the first superconducting coil but supplies a current to the second superconducting coil, a first detector that detects quenching of the first superconducting coil, and a control unit that performs first adjustment processing of adjusting a current to be supplied from the second power source to the second superconducting coil in response to detection of quenching of the first superconducting coil by the first detector.

## Description

### Technical Field

The present invention relates to a superconducting magnet device, a particle beam therapy system, and a method of controlling a superconducting magnet device.

### Background Art

Particle beam therapy has been used in the past to treat patients for malignant tumors such as cancer by irradiating the malignant tumors with a charged particle beam accelerated to a high energy. A device used in particle beam therapy may, in order to emit a charged particle beam at an arbitrary angle to the isocenter inside a treatment room, deflect the charged particle beam by using an electromagnet capable of generating a strong magnetic field. In addition, using superconducting coils for the electromagnet has been proposed (e.g., Patent Documents 1 to 3).

### Citation List

### Patent Literature

Patent Document 1: JP 2021-159267 A
Patent Document 2: JP 2017-162896 A
Patent Document 3: JP 2021-93440 A

### Summary of Invention

### Technical Problem

Superconducting coils may experience quenching in which the superconducting coils lose their superconductive state and are brought into a normal conductive state. When a superconducting coil experiences quenching, the magnetic flux density of the superconducting coil is changed and an eddy current is generated, and thereby, another superconducting coil may generate heat or the power source supplying a current to the coil may generate an induced electromotive force, causing an overvoltage. As described above, quenching of a superconducting coil may affect other superconducting coils and peripheral apparatuses. As a result, it may take time to recover a device.

An embodiment of the present invention provides a technique for reducing the influence of quenching of a superconducting coil on other components.

### Solution to Problem

The present invention includes the following aspects.

### [Aspect 1]

A superconducting magnet device including a first superconducting coil, a second superconducting coil, a first power source that supplies a current to the first superconducting coil and does not supply a current to the second superconducting coil, a second power source that does not supply a current to the first superconducting coil but supplies a current to the second superconducting coil, a first detector that detects quenching of the first superconducting coil, and a control unit that performs first adjustment processing of adjusting a current to be supplied from the second power source to the second superconducting coil in response to detection of quenching of the first superconducting coil by the first detector.

### [Aspect 2]

The superconducting magnet device described in aspect 1, in which the control unit reduces the current to be supplied to the second superconducting coil by the second power source in the first adjustment processing to be lower than a current before the first adjustment processing is performed.

### [Aspect 3]

The superconducting magnet device described in any one of aspect 1 and 2, in which the control unit sets the current to be supplied to the second superconducting coil by the second power source in the first adjustment processing to 0 [A].

### [Aspect 4]

The superconducting magnet device described in any one of aspects 1 to 3 further including a first breaker that interrupts a current supplied from the first power source to the first superconducting coil, in which the control unit causes the first breaker to interrupt the current supplied to the first superconducting coil when the first detector detects quenching of the first superconducting coil.

### [Aspect 5]

The superconducting magnet device described in any one of aspects 1 to 4 further including a second detector that detects quenching of the second superconducting coil, and a second breaker that interrupts a current supplied from the second power source to the second superconducting coil, in which the control unit causes the second breaker to interrupt the current supplied to the second superconducting coil when the second detector detects quenching of the second superconducting coil during the first adjustment processing.

### [Aspect 6]

The superconducting magnet device described in aspect 5, in which the control unit performs the first adjustment processing in response to quenching of the first superconducting coil detected by the first detector and a temperature of the second superconducting coil being equal to or higher than a first threshold value that is lower than a superconducting transition temperature of the second superconducting coil.

### [Aspect 7]

The superconducting magnet device described in any one of aspects 5 and 6, in which the control unit performs the first adjustment processing in response to quenching of the first superconducting coil detected by the first detector and a temperature gradient of the second superconducting coil being equal to or greater than a predetermined value.

### [Aspect 8]

The superconducting magnet device described in any one of aspects 1 to 7, in which the control unit ends the first adjustment processing when a temperature of the first superconducting coil experiencing quenching becomes equal to or lower than a second threshold value.

### [Aspect 9]

The superconducting magnet device described in any one of aspects 1 to 8 further including a cryostat that accommodates the first superconducting coil and the second superconducting coil.

### [Aspect 10]

The superconducting magnet device described in aspect 9 further including a heat transfer mechanism that thermally connects the first superconducting coil and the second superconducting coil in the cryostat.

### [Aspect 11]

The superconducting magnet device described in any one of aspects 9 and 10 further including a third superconducting coil accommodated in the cryostat, and a third power source that does not supply a current to the first superconducting coil and the second superconducting coil but supplies a current to the third superconducting coil, in which the second superconducting coil, the first superconducting coil, and the third superconducting coil are provided side by side in this order in the cryostat, and the control unit also performs second adjustment processing of adjusting a current to be supplied to the third superconducting coil from the third power source in response to detection of quenching of the first superconducting coil by the first detector.

### [Aspect 12]

The superconducting magnet device described in any one of aspects 9 and 10 further including a third superconducting coil accommodated in the cryostat, and a third power source that does not supply a current to the first superconducting coil and the second superconducting coil but supplies a current to the third superconducting coil, in which the first superconducting coil, the second superconducting coil, and the third superconducting coil are provided side by side in this order in the cryostat, and the control unit also performs second adjustment processing of adjusting a current to be supplied to the third superconducting coil from the third power source in response to detection of quenching of the first superconducting coil by the first detector.

### [Aspect 13]

The superconducting magnet device described in any one of aspects 9 and 10 further including a third superconducting coil accommodated in a cryostat that is different from the cryostat, in which the first superconducting coil and the second superconducting coil are accommodated, and
a third power source that does not supply a current to the first superconducting coil and the second superconducting coil but supplies a current to the third superconducting coil, in which the second superconducting coil, the first superconducting coil, and the third superconducting coil are provided side by side in this order, and the control unit also performs second adjustment processing of adjusting a current to be supplied to the third superconducting coil from the third power source in response to detection of quenching of the first superconducting coil by the first detector.

### [Aspect 14]

The superconducting magnet device described in any one of aspect 11 or 13, in which the control unit is capable of performing a first control pattern in which the first power source and the second power source supply currents to the first superconducting coil and the second superconducting coil, respectively, and a second control pattern in which the first power source and the third power source supply currents to the first superconducting coil and the third superconducting coil, respectively, the control unit performs the first adjustment processing when the first detector detects quenching of the first superconducting coil during the first control pattern, and the control unit performs the second adjustment processing when the first detector detects quenching of the first superconducting coil during the second control pattern.

### [Aspect 15]

The superconducting magnet device described in any one of aspects 1 to 14, in which the first superconducting coil and the second superconducting coil are adjacent to each other.

### [Aspect 16]

The superconducting magnet device described in any one of aspects 1 to 15, in which the first detector detects quenching of the first superconducting coil by detecting a voltage of the first superconducting coil.

### [Aspect 17]

A particle beam therapy system including the superconducting magnet device described in any one of aspects 1 to 16, an accelerator that generates and emits a charged particle beam, and a beam transport system that transports a charged particle beam emitted from the accelerator to the superconducting magnet device.

### [Aspect 18]

The particle beam therapy system described in aspect 17, in which the accelerator does not emit a charged particle beam while the control unit performs current adjustment.

### [Aspect 19]

A method of controlling a superconducting magnet device including a first superconducting coil, a second superconducting coil, a first power source that supplies a current to the first superconducting coil and does not supply a current to the second superconducting coil, and a second power source that does not supply a current to the first superconducting coil but supplies a current to the second superconducting coil, the method including detecting quenching of the first superconducting coil, and adjusting a current to be supplied from the second power source to the second superconducting coil in response to detection of quenching of the first superconducting coil in the detecting.

### Advantageous Effects of Invention

According to an embodiment of the present invention, it is possible to reduce the influence of quenching of a superconducting coil on other components.

### Brief Description of Drawings

FIG. 1 is a schematic diagram of a superconducting magnet device according to an embodiment.
FIG. 2 is a flowchart showing a processing example of a control unit.
FIG. 3 is a diagram showing temporal changes in current and temperature of superconducting coils when a superconducting coil is experiencing quenching.
FIG. 4 is a schematic diagram of a superconducting magnet device according to an embodiment.
FIG. 5 is a flowchart showing a processing example of a control unit.
FIG. 6 is a diagram showing temporal changes in temperature of superconducting coils when a superconducting coil is experiencing quenching.
FIG. 7 is a flowchart showing a processing example of a control unit.
FIG. 8 is a flowchart showing a processing example of a control unit.
FIG. 9 is an apparatus configuration diagram of a particle beam therapy system according to an embodiment.

### Description of Embodiments

### <First Embodiment>

### <Configuration of Superconducting Magnet Device>

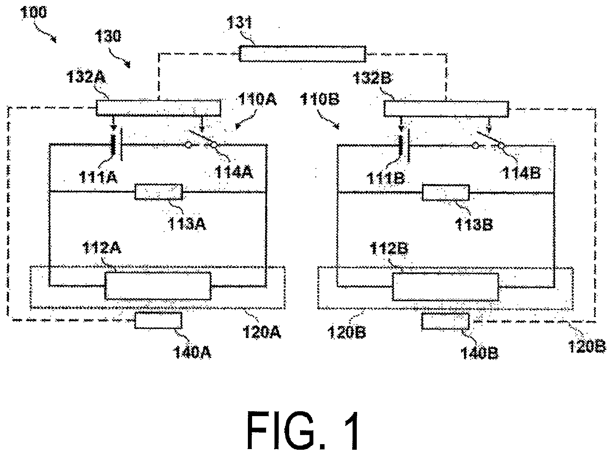

FIG. 1 is a schematic diagram of a superconducting magnet device 100 according to an embodiment. For example, the superconducting magnet device 100 is a device capable of generating a strong magnetic field in order to emit a charged particle beam at an arbitrary angle to the isocenter inside a treatment room in particle beam therapy. The superconducting magnet device 100 includes circuits 110A and 110B, cryostats 120A and 120B, and a control unit 130.

The circuit 110A includes a power source 111A, a superconducting coil 112A, a protective resistor 113A, and a breaker 114A. The power source 111A supplies a current to the superconducting coil 112A. The superconducting coil 112A generates a magnetic field in accordance with the current supplied from the power source 111A. The superconducting magnet device 100 deflects a charged particle beam by using the magnetic field. Although not illustrated, a core may be incorporated inside the superconducting coil 112A. The protective resistor 113A attenuates a current flowing through the superconducting coil 112A when the superconducting coil 112A experiences quenching. The protective resistor 113A is connected to the power source 111A in tandem with the superconducting coil 112A. The breaker 114A interrupts a current flowing from the power source 111A to the superconducting coil 112A. The breaker 114A is connected to the power source 111A in series with the superconducting coil 112A.

The cryostat 120A is an insulated cooler that maintains the superconducting coil 112A at an extremely low temperature and accommodates the superconducting coil 112A. Since the superconducting coil 112A is kept in an extremely low temperature state by the cryostat 120A, the superconducting coil 112A can maintain a superconductive state. A coolant, for example, helium gas, liquid helium, or liquid nitrogen is used for the cryostat 120A.

The control unit 130 controls operations of the superconducting magnet device 100. The control unit 130 includes an integrated control unit 131 and power source control units 132A and 132B. The integrated control unit 131 integrally controls the entire superconducting magnet device 100. The power source control unit 132A controls the power source 111A and the breaker 114A in the circuit 110A to adjust a current flowing to the superconducting coil 112A.

Each of the integrated control unit 131 and the power source control units 132A and 132B may be a control circuit including one or more processors such as central processing units (CPUs) and memories. These functions may be realized by a processor operating according to a program (software) readably stored in a memory. The program may be stored in, for example, a storage device inside the superconducting magnet device 100.

Note that each of the integrated control unit 131 and the power source control units 132A and 132B may be configured to include hardware such as integrated circuits represented by a field programmable gate array (FPGA) or an application specific integrated circuit (ASIC) instead of or in addition to the processors and memories.

In addition, here, an aspect in which the integrated control unit 131 and the power source control units 132A and 132B are separate control circuits is exemplified. However, the control unit 130 may be configured by a single control circuit as described above to realize the functions of the integrated control unit 131 and the power source control units 132A and 132B.

A detector 140A detects quenching that has occurred in the superconducting coil 112A. In the present embodiment, the detector 140A detects a voltage of the superconducting coil 112A. The detection result from the detector 140A is output to the power source control unit 132A. When the voltage of the superconducting coil 112A detected by the detector 140A is equal to or higher than a predetermined value, the power source control unit 132A may determine that the superconducting coil 112A is experiencing quenching. Note that, as a method for the detector 140A to detect quenching of the superconducting coil 112A, a known technique can be appropriately used. For example, the detector 140A may detect a temperature of the superconducting coil 112A. In this case, when the temperature of the superconducting coil 112A detected by the detector 140A is equal to or higher than a superconducting transition temperature, the power source control unit 132A may determine that the superconducting coil 112A is experiencing quenching.

A circuit 110B, a power source 111B, a superconducting coil 112B, a protective resistor 113B, a breaker 114B, a cryostat 120B, the power source control unit 132B, and a detector 140B are the same as the circuit 110A, the power source 111A, the superconducting coil 112A, the protective resistor 113A, the breaker 114A, the cryostat 120A, the power source control unit 132A, and the detector 140A, respectively, and thus description thereof is omitted.

In the present embodiment, the superconducting magnet device 100 includes the two circuits 110A and 110B. In addition, the power source 111A included in the circuit 110A supplies a current to the superconducting coil 112A likewise included in the circuit 110A, but supplies no current to the superconducting coil 112B included in the circuit 110B. On the other hand, the power source 111B included in the circuit 110B supplies a current to the superconducting coil 112B likewise included in the circuit 110B, but supplies no current to the superconducting coil 112A included in the circuit 110A. In other words, the superconducting coil 112A and the superconducting coil 112B are not connected to the power source 111A or the power source 111B either in series or in parallel. In other words, it can be said that the superconducting coil 112A and the superconducting coil 112B are in an independent relationship in terms of electric circuit. Furthermore, in the present embodiment, the superconducting coil 112B is adjacent to the superconducting coil 112A.

### <Operation When Quenching Occurs>

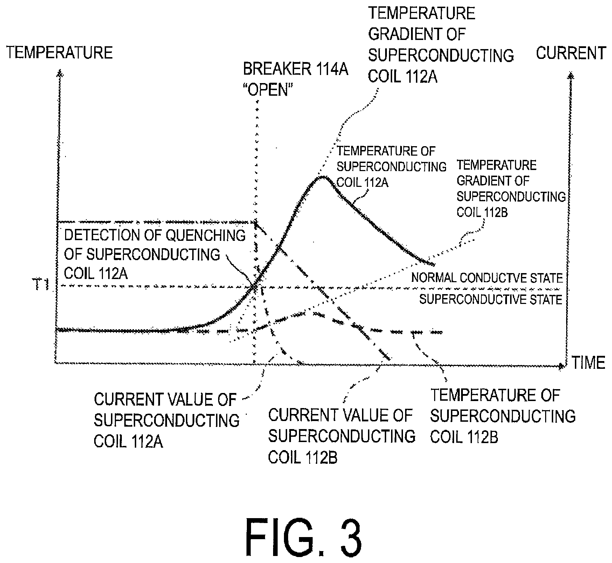

Next, the operation on the circuit 110A side when quenching occurs in the superconducting coil 112A will be described. Upon determining that the superconducting coil 112A is experiencing quenching, the power source control unit 132A causes the breaker 114A to operate to interrupt the supply of a current from the power source 111A to the superconducting coil 112A. Then, the circuit 110A turns into a closed circuit of the superconducting coil 112A and the protective resistor 113A. Thus, the current flowing through the superconducting coil 112A is attenuated. Thereafter, when the temperature of the superconducting coil 112A decreases with time and becomes lower than a temperature T1 (see FIG. 3) which is the superconducting transition temperature, the superconducting coil 112A can return to the superconductive state.

However, when a plurality of superconducting coils 112A and 112B are provided as in the present embodiment, a change in the state of one superconducting coil may affect the other superconducting coil. For example, when the superconducting coil 112A experiences quenching, the magnetic flux density of the superconducting coil 112A changes due to a change in the current of the superconducting coil 112A, which may cause the superconducting coil 112B to be quenched. In addition, due to the change in the magnetic flux density of the superconducting coil 112A, an induced electromotive force may be generated in the superconducting coil 112B by electromagnetic induction. Then, the induced electromotive force causes the superconducting coil 112B to be overvoltage, and the power source 111B connected to the superconducting coil 112B may break down. Thus, quenching of the superconducting coil may affect other components of the device. As a result, when the superconducting coil is quenched, it may take time to recover until the device can perform a predetermined operation again (for example, an operation of emitting a charged particle beam at an arbitrary angle in particle beam therapy). Therefore, in the present embodiment, control performed as described below can reduce the influence of quenching of the superconducting coil on other components.

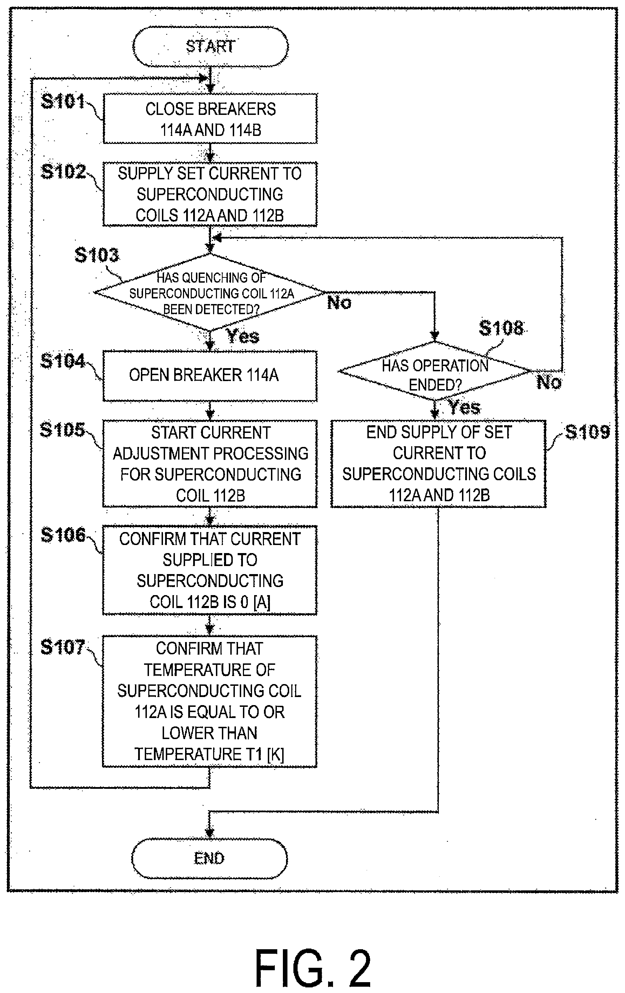

FIG. 2 is a flowchart showing a processing example of the control unit 130. For example, this flowchart is executed while the superconducting magnet device 100 is performing a predetermined operation such as an operation of generating a strong magnetic field in order to emit a charged particle beam at an arbitrary angle in particle beam therapy.

In S101, the control unit 130 closes the breakers 114A and 114B so that the current can be supplied from the power sources 111A and 111B to the superconducting coils 112A and 112B. To be specific, the integrated control unit 131 instructs the power source control unit 132A to close the breaker 114A, and instructs the power source control unit 132B to close the breaker 114B. Upon receiving the instruction, the power source control units 132A and 132B close the breakers 114A and 114B, respectively. Note that when the breakers 114A and 114B are already closed, the control unit 130 maintains the state.

In S102, the control unit 130 supplies a set current to the superconducting coils 112A and 112B. To be specific, the power source control units 132A and 132B that have received an instruction from the integrated control unit 131 control the power sources 111A and 111B so that set currents are supplied to the superconducting coils 112A and 112B, respectively. Note that the set current of the superconducting coil 112A may be the same as or different from the set current of the superconducting coil 112B.

In S103, the control unit 130 checks whether quenching of the superconducting coil 112A has been detected, and proceeds to S104 if quenching has been detected and proceeds to S108 if quenching has not been detected. The power source control unit 132A checks whether quenching has occurred in the superconducting coil 112A based on the detection result of the detector 140A. In addition, when the detector 140A detects quenching of the superconducting coil 112A, the power source control unit 132A transmits information indicating that quenching of the superconducting coil 112A has been detected to the integrated control unit 131.

In S104, the control unit 130 opens the breaker 114A. As a result, the supply of the current from the power source 111A to the superconducting coil 112A is interrupted, and the circuit 110A becomes a closed circuit of the superconducting coil 112A and the protective resistor 113A. Thus, the current flowing through the superconducting coil 112A is attenuated, and the energy accumulated in the superconducting coil 112A is consumed by the protective resistor 113A. Therefore, the quenched superconducting coil 112A can be protected from damage or the like.

In S105, the control unit 130 starts current adjustment processing for the superconducting coil 112B. When receiving the information indicating that the quenching of the superconducting coil 112A has been detected from the power source control unit 132A, the integrated control unit 131 instructs the power source control unit 132B to perform current adjustment for the superconducting coil 112B. The power source control unit 132B having received the instruction adjusts the current supplied from the power source 111B to the superconducting coil 112B. In the present embodiment, the power source control unit 132B adjusts the current supplied to the superconducting coil 112B by controlling the current output from the power source 111B. To be more specific, the power source control unit 132B adjusts the current supplied to the superconducting coil 112B by the power source 111B to 0 [A] so that the current supplied to the superconducting coil 112B becomes 0 [A]. That is, the current supplied to the superconducting coil 112B is adjusted with the breaker 114B closed and the power source 111B and the superconducting coil 112B connected to each other in a circuit. Note that the current adjustment processing (S105) for the superconducting coil 112B may be performed simultaneously with the opening operation (S104) of the breaker 114A or as soon as possible after the opening operation of the breaker 114A.

Note that, although the power source control unit 132B sets the current supplied from the power source 111B to the superconducting coil 112B to 0 [A] in the current adjustment processing in the present embodiment, another mode may be adopted. For example, the power source control unit 132B may set the current supplied from the power source 111B to the superconducting coil 112B in the current adjustment processing to a predetermined current (> 0 [A]) that is lower than the set current. Also in this mode, it is possible to curb a temperature rise of the superconducting coil 112B as compared with the case in which the current adjustment processing is not performed. In addition, by setting the current supplied to the superconducting coil 112B larger than 0 [A], the time required for recovery of the device can be shortened.

Here, it is also considered that, when the superconducting coil 112A experiences quenching, the current supplied to the superconducting coil 112B is maintained. However, in such a case, there is concern that the power source 111B and the superconducting coil 112B may become an overvoltage due to the influence of induced electromotive forces caused by electromagnetic induction of the superconducting coil 112A, leading to breakdown of the power source 111B. On the other hand, in the present embodiment, since the current supplied to the superconducting coil 112B is adjusted when the superconducting coil 112A experiences quenching, the influence of an overvoltage resulting from electromagnetic induction caused by quenching of the superconducting coil 112A can be reduced.

In S106, the control unit 130 confirms that the current supplied to the superconducting coil 112B is 0 [A]. Then, in S107, the control unit 130 confirms that the temperature of the superconducting coil 112A is equal to or lower than the temperature T1 [K]. Here, the temperature T1 may be the superconducting transition temperature of a material constituting the superconducting coil 112A. In addition, for example, the temperature T1 may be a temperature lower than the superconducting transition temperature. Thereafter, the control unit 130 returns to S101. That is, the control unit 130 ends the current adjustment processing for the superconducting coil 112B and supplies the set current to the superconducting coil 112A again and to the superconducting coil 112B.

On the other hand, when the processing proceeds from S103 to S108, the control unit 130 checks whether the predetermined operation has ended, proceeds to S109 if the predetermined operation has ended, and returns to S103 if the predetermined operation has not ended. The predetermined operation is, for example, an operation in which the superconducting magnet device 100 generates a strong magnetic field in order to emit a charged particle beam at an arbitrary angle in particle beam therapy.

In S109, the control unit 130 ends the supply of the set current to the superconducting coils 112A and 112B.

FIG. 3 is a diagram showing temporal changes in current and temperature of the superconducting coils 112A and 112B when the superconducting coil 112A is experiencing quenching. Before quenching occurs, the temperatures of the superconducting coils 112A and 112B are lower than the superconducting transition temperature (temperature T1).

When quenching occurs in the superconducting coil 112A, the temperature of the superconducting coil 112A starts to rise. Thereafter, when the detector 140A detects the quenching of the superconducting coil 112A, the breaker 114A is opened (S104 in FIG. 2). The temperature of the superconducting coil 112A temporarily rises even after the breaker 114A is opened. This is because an induced current is generated due to a change in magnetic flux density associated with current interruption, and as a result, large AC loss takes place, and a temperature gradient which is a temperature change of the superconducting coil 112A per unit time becomes large. Thereafter, however, the energy stored in the superconducting coil 112A gradually decreases as it is consumed by the protective resistor 113A.

On the other hand, in the superconducting coil 112B, the current supplied through the current adjustment by the control unit 130 decreases in accordance with the occurrence of quenching in the superconducting coil 112A (S105 in FIG. 2). As a result, even if the temperature of the superconducting coil 112B rises under the influence of a change in the magnetic flux density of the superconducting coil 112A or the like, the temperature gradient of the superconducting coil 112B becomes gentle. Therefore, the current supplied to the superconducting coil 112B can be reduced while maintaining the superconductive state. Therefore, the occurrence of quenching in the superconducting coil 112B and damage to the power source 111B and the superconducting coil 112B caused by an overvoltage can be prevented.

Here, when the superconducting coil 112A experiences quenching, the breaker 114B may also be opened for the superconducting coil 112B to interrupt the current from the power source 111B. In this case, although damage to the power source 111B and the superconducting coil 112B can be prevented, there is a possibility that a current may flow through the protective resistor 113B, causing the temperature of the superconducting coil 112B to increase, and even the superconducting coil 112B may experience quenching. As a result, it may take more time for both the superconducting coil 112A and the superconducting coil 112B to return to the superconductive state. On the other hand, in the present embodiment, damage to the power source 111B and the superconducting coil 112B due to an overvoltage can be prevented while curbing the occurrence of quenching in the superconducting coil 112B as described above.

Note that, in the present embodiment, the process of adjusting the current of the superconducting coil 112B when quenching occurs in the superconducting coil 112A has been described. In parallel with this process, a process of adjusting the current of the superconducting coil 112A when quenching occurs in the superconducting coil 112B may be performed.

### <Second Embodiment>

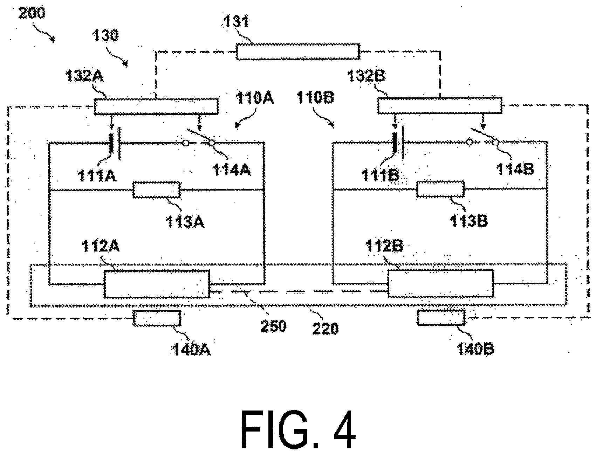

FIG. 4 is a schematic diagram of a superconducting magnet device 200 according to an embodiment. The superconducting magnet device 200 of the present embodiment is different from the superconducting magnet device 100 of the first embodiment mainly in that two superconducting coils are accommodated in the same cryostat. Hereinafter, the same elements as those of the superconducting magnet device 100 according to the first embodiment are denoted by the same reference numerals, and description thereof is appropriately omitted. In addition, modifications and the like described for the superconducting magnet device 100 can also be appropriately employed in the superconducting magnet device 200.

In the superconducting magnet device 200, superconducting coils 112A and 112B are accommodated in the same cryostat 220. The superconducting coil 112A and the superconducting coil 112B are thermally connected to each other by a heat transfer mechanism 250. As the heat transfer mechanism 250, for example, a plate or a braided wire made of aluminum or the like having high thermal conductivity properties at a low temperature may be used. Since the superconducting coils 112A and 112B are thermally connected, the superconducting coils can be uniformly cooled, enhancing cooling efficiency. Note that, if the superconducting coils 112A and 112B are accommodated in the same cryostat 220, a configuration without the heat transfer mechanism 250 may be employed. Note that, even when the heat transfer mechanism 250 is omitted, it can be said that the superconducting coils 112A and 112B of the superconducting magnet device 200 are in a thermally-connected relationship since the superconducting coils are accommodated in the same cryostat 220. On the other hand, since the superconducting coils 112A and 112B of the superconducting magnet device 100 are accommodated in the separate cryostats 120A and 120B, respectively it can be said that the superconducting coils are in a thermally-independent relationship.

As a process performed when quenching occurs in the superconducting coil 112A, the superconducting magnet device 200 may adopt a process similar to the flowchart of FIG. 2. In the superconducting magnet device 200, the superconducting coils 112A and 112B are accommodated in the same cryostat 220 and thermally connected. For this reason, the temperature of the superconducting coil 112B is more likely to increase due to the occurrence of quenching in the superconducting coil 112A in the superconducting magnet device 200 than in the superconducting magnet device 100 of the first embodiment. Even in such a case, the occurrence of quenching in the superconducting coil 112B can be prevented while curbing an overvoltage of the power source 111B and the superconducting coil 112B due to electromagnetic induction caused by quenching of the superconducting coil 112A.

### <Third Embodiment>

In the superconducting magnet device 100 of the first embodiment or the superconducting magnet device 200 of the second embodiment, a process different from the process shown in the flowchart of FIG. 2 can be performed as the process when quenching occurs in the superconducting coil 112A. Hereinafter, another example of the process when quenching occurs in the superconducting coil 112A in the superconducting magnet device 100 or the superconducting magnet device 200 will be described.

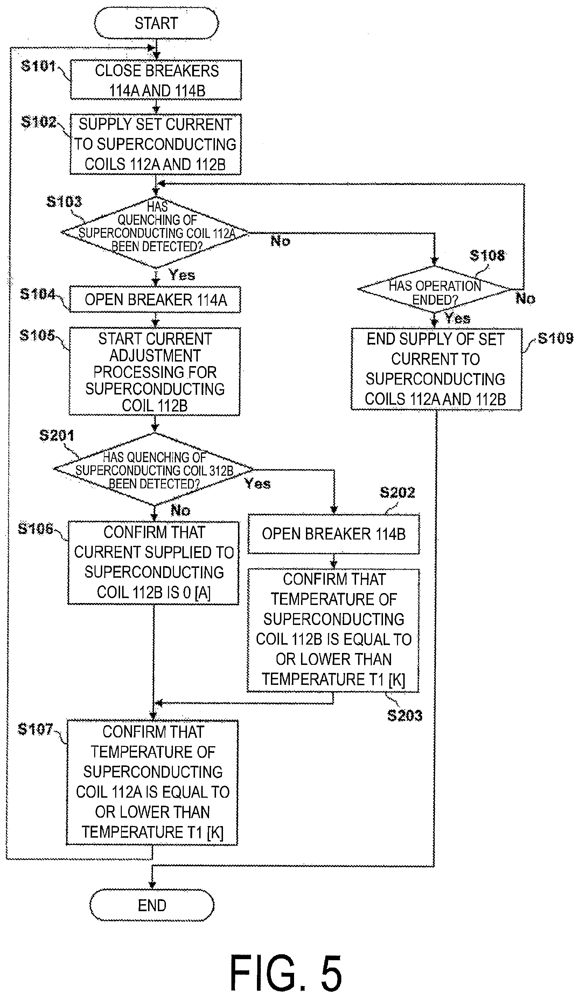

FIG. 5 is a flowchart showing a processing example of the control unit 130. In the flowchart of FIG. 2, current adjustment processing for the superconducting coil 112B is performed when quenching occurs in the superconducting coil 112A. On the other hand, depending on the positional relationship between the superconducting coil 112A and the superconducting coil 112B as well as the temperature state of the superconducting coil 112A, it is conceivable that quenching could occur in the superconducting coil 112B during current adjustments of the superconducting coil 112B. Therefore, in this flowchart, the breaker 114B interrupts the current when even the superconducting coil 112B experiences quenching. Note that the same processes as those in the flowchart of FIG. 2 are denoted by the same reference numerals, and description thereof is omitted as appropriate.

In this flowchart, the control unit 130 proceeds to S201 after S105. That is, similarly to the flowchart of FIG. 2, when quenching of the superconducting coil 112A is detected (S103: Yes), the breaker 114A is opened (S104), and the current adjustment processing for the superconducting coil 112B is performed (S105). Then in S105, the control unit 130 checks whether quenching of the superconducting coil 112B has been detected, and proceeds to S202 if quenching has been detected and proceeds to S106 if quenching has not been detected. The control unit 130 checks whether quenching has occurred in the superconducting coil 112B based on the detection result of the detector 140B.

When quenching of the superconducting coil 112B has not been detected, the subsequent processing is the same as the processing of the flowchart of FIG. 2 (S106 and S107).

On the other hand, when quenching of the superconducting coil 112B has been detected, the control unit 130 opens the breaker 114B in S202. That is, the control unit 130 causes the breaker 114B to forcibly interrupt the current flowing through the superconducting coil 112B. Thereafter, in S203, the control unit 130 confirms that the temperature of the superconducting coil 112B is equal to or lower than the temperature T1 [K], and proceeds to S107.

According to the processing described above, when quenching occurs in the superconducting coil 112A, the control unit 130 performs the current adjustment processing for the superconducting coil 112B (S105). Then, when a temperature rise in the superconducting coil 112B can be prevented through the current adjustment processing, the circuit 310A and the breaker 114A are closed after confirming that the temperature of the superconducting coil 112A has dropped to the temperature T1 [K]. As a result, the superconducting coil 112A can be recovered from the occurrence of quenching without causing quenching in the superconducting coil 112B. Therefore, it is possible to shorten the time from the occurrence of quenching in the superconducting coil 112A to recovery therefrom.

On the other hand, even if the current adjustment processing for the superconducting coil 112B is performed when quenching has occurred in the superconducting coil 112A, the superconducting coil 112B may experience quenching. In such a case, by opening the breaker 114B to interrupt the current supplied to the superconducting coil 112B (S202), it is possible to reduce an impact such as damage or the like caused by quenching in the superconducting coil 112B.

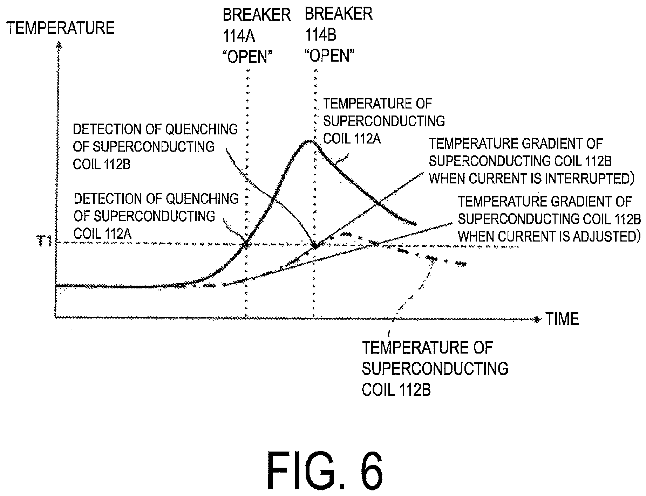

FIG. 6 is a diagram showing temporal changes in the temperature of the superconducting coils 112A and 112B when the superconducting coil 112A is experiencing quenching. Before quenching occurs, the temperatures of the superconducting coils 112A and 112B are lower than the superconducting transition temperature (temperature T1).

When quenching occurs in the superconducting coil 112A, the temperature of the superconducting coil 112A starts to rise. Thereafter, when the detector 140A detects the quenching of the superconducting coil 112A, the breaker 114A is opened (S104 in FIG. 5). The temperature of the superconducting coil 112A temporarily rises even after the breaker 114A is opened. This is because an induced current is generated due to a change in magnetic flux density associated with current interruption, and as a result, large AC loss takes place, and a temperature gradient which is a temperature change of the superconducting coil 112A per unit time becomes large. Thereafter, however, the energy stored in the superconducting coil 112A gradually decreases as it is consumed by the protective resistor 113A.

On the other hand, as the temperature of the superconducting coil 112A thermally connected to the superconducting coil 112B rises, the temperature of the superconducting coil 112B also rises. In this example, although the temperature gradient of the superconducting coil 112B at the time of the temperature rise is reduced due to the current adjustment processing (S105 in FIG. 5), the temperature of the superconducting coil 112B exceeds the superconducting transition temperature (temperature T1). For this reason, the detector 140B detects the quenching of the superconducting coil 112B (S201 in FIG. 5). Thereafter, since the breaker 114B is opened to let a current flow through the protective resistor 113B, the temperature gradient at the time of the temperature rise temporarily increases, but the temperature of the superconducting coil 112B gradually decreases as the protective resistor consumes the accumulated energy.

In the present embodiment, when the superconducting coil 112A experiences quenching, the current adjustment processing is first performed for the superconducting coil 112B, and when the superconducting coil 112B experiences quenching even after the current adjustment processing is performed, the breaker 114B is opened. In this way, by performing the processing on the superconducting coil 112B in stages, it is possible to achieve shortening of the time from the occurrence of quenching of the superconducting coil 112A to the recovery therefrom and protection of the superconducting coil 112B in a compatible manner.

### <Fourth Embodiment>

Hereinafter, another processing example when quenching occurs in the superconducting coil 112A in the superconducting magnet device 100 or the superconducting magnet device 200 will be described. In the present processing example, when the superconducting coil 112A is quenched, the current adjustment processing for the superconducting coil 112B is not immediately performed, and a set current of the superconducting coil 112B is maintained under a certain situation. By maintaining the set current of the superconducting coil 112B, when the superconducting coil 112A recovers from the quenched state to the superconductive state relatively earlier, the recovery of the device can be expedited.

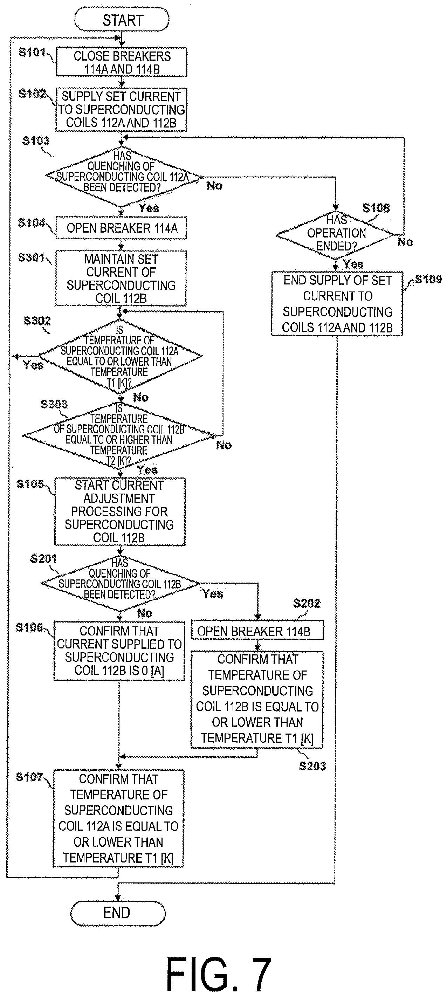

FIG. 7 is a flowchart showing a processing example of the control unit 130. Note that the same processes as those in the flowcharts of FIG. 2 or 5 are denoted by the same reference numerals, and description thereof is omitted as appropriate.

In this flowchart, the control unit 130 proceeds to S301 after S104. In S301, the control unit 130 maintains the set current of the superconducting coil 112B.

In S302, the control unit 130 checks whether the temperature of the superconducting coil 112A is equal to or lower than T1 [K], and returns to S101 if the temperature is equal to or lower than T1 [K], or returns to S303 if not. Then, in S303, the control unit 130 checks whether the temperature of the superconducting coil 112B is equal to or lower than a temperature T2 [K], and proceeds to S105 if the temperature is equal to or higher than the temperature T2 [K], and proceeds to the S302 if not. Here, the temperature T2 is a predetermined temperature lower than the temperature T1 which is the superconducting transition temperature (T2 < T1). The processing after S105 is the same as that in the flowchart of FIG. 5.

According to this processing example, even when the superconducting coil 112A experiences quenching, the control unit 130 maintains the set current of the superconducting coil 112B (S301) when the superconducting coil 112B is at a temperature equal to or lower than the temperature T2 (< temperature T1). Thus, when the superconducting coil 112A returns from the normal conductive state to the superconductive state while the set current of the superconducting coil 112B is maintained, the recovery of the device can be expedited.

On the other hand, when the temperature of the superconducting coil 112B exceeds the temperature T2 while the set current of the superconducting coil 112B is maintained, it is possible to curb the temperature rise of the superconducting coil 112B by performing the current adjustment processing for the superconducting coil 112B (S105). Furthermore, when the temperature of the superconducting coil 112B exceeds the temperature T1 and the superconducting coil 112B experiences quenching even if the current adjustment processing for the superconducting coil 112B is performed, the breaker 114B can be opened to protect the superconducting coil 112B.

### <Fifth Embodiment>

Hereinafter, another processing example when quenching occurs in the superconducting coil 112A in the superconducting magnet device 100 or the superconducting magnet device 200 will be described. The present processing example is similar to that in the fourth embodiment in that, when the superconducting coil 112A is quenched, the current adjustment processing for the superconducting coil 112B is not immediately performed, and a set current of the superconducting coil 112B is maintained under a certain situation. On the other hand, in the present embodiment, it is determined whether the current adjustment processing for the superconducting coil 112B is to be performed in consideration of the temperature gradient of the superconducting coil 112B. As a result, the current adjustment processing for the superconducting coil 112B can be performed more appropriately.

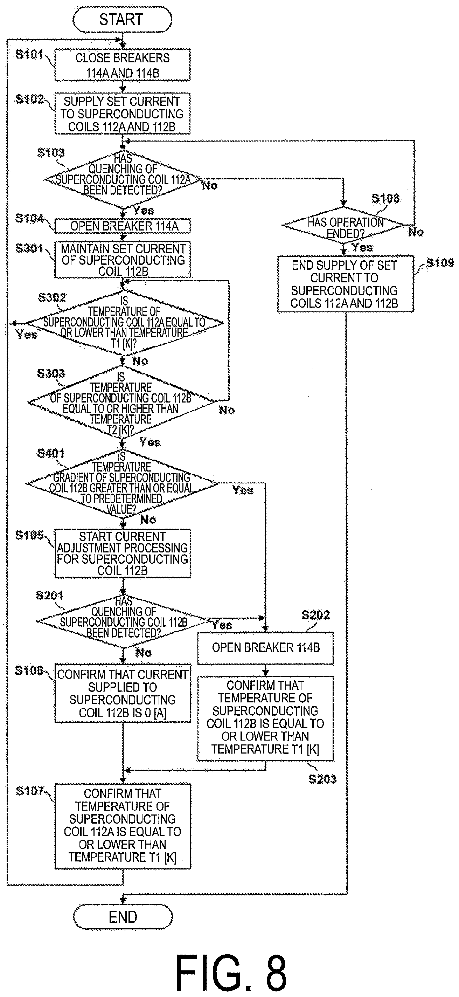

FIG. 8 is a flowchart showing a processing example of the control unit 130. Note that the same processes as those in the flowcharts of FIG. 2, 5, or 7 are denoted by the same reference numerals, and description thereof is omitted as appropriate.

In this flowchart, when the temperature of the superconducting coil 112B is equal to or higher than the temperature T2 [K] in S303, the control unit 130 proceeds to S401. In S401, the control unit 130 checks whether the temperature gradient (dT/dt) of the superconducting coil 112B is greater than a predetermined value, and proceeds to S202 to open the breaker 114B if the temperature gradient is greater than or equal to the predetermined value, or proceeds to S105 if not. The processing thereafter is the same as that in the flowchart of FIG. 7.

Although the predetermined value of the temperature gradient can be set as appropriate, for example, the predetermined value may be a positive value. In other words, when the temperature of the superconducting coil 112B is equal to or higher than the temperature T2 and the temperature gradient is equal to or higher than a predetermined value (> 0), the control unit 130 may open the breaker 114B without performing the current adjustment processing for the superconducting coil 112B. As a result, when the temperature gradient is such that the temperature of the superconducting coil 112B is likely to exceed the temperature T1 even if the current adjustment processing for the superconducting coil 112B is performed, the breaker 114B is quickly opened to prevent damage to the superconducting coil 112B. On the other hand, the control unit 130 may perform the current adjustment processing for the superconducting coil 112B in the case of a temperature gradient at which the temperature of the superconducting coil 112B is likely to be maintained below the temperature T1 when the current adjustment processing for the superconducting coil 112B is performed. As a result, when the superconducting coil 112A returns from the normal conductive state to the superconductive state, the recovery of the device can be expedited.

Note that, in the flowchart of FIG. 8, the current supplied to the superconducting coil 112B and the operation of the breaker 114B are controlled based on the temperature and the temperature gradient of the superconducting coil 112B. However, the current supplied to the superconducting coil 112B and the operation of the breaker 114B may be controlled based on only the temperature gradient of the superconducting coil 112B. For example, in the flowchart of FIG. 7, the conditional branch of 401 in FIG. 8 may be employed instead of the conditional branch of S303.

### <Sixth Embodiment>

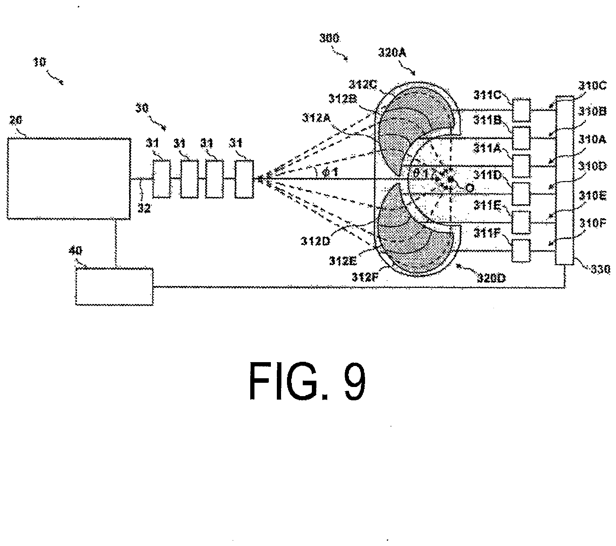

FIG. 9 is an apparatus configuration diagram of a particle beam therapy system 10 according to an embodiment. The particle beam therapy system 10 is a device that has been used to perform beam therapy to treat patients for malignant tumors such as cancer by irradiating the malignant tumors with a charged particle beam accelerated to a high energy. The particle beam therapy system 10 includes an accelerator 20, a beam transport system 30, an acceleration control unit 40, and the superconducting magnet device 300.

The accelerator 20 is a device that generates and emits charged particle beams, and is, for example, a synchrotron, a cyclotron, or a linear accelerator. A charged particle beam generated by the accelerator 20 is guided to the superconducting magnet device 300 through the beam transport system 30.

The beam transport system 30 transports the charged particle beam generated by the accelerator 20 to the superconducting magnet device 300. The beam transport system 30 includes a beam adjustment unit 31 and a vacuum duct 32.

The beam adjustment unit 31 adjusts a shape, a dose, a traveling direction, and the like of the charged particle beam. The beam adjustment unit 31 includes a beam slit for adjusting a beam shape and/or a dose, an electromagnet for adjusting a travel direction of the charged particle beam, a quadrupole electromagnet for adjusting the beam shape of the charged particle beam, a steering electromagnet for finely adjusting a beam position of the charged particle beam, and the like as appropriate in accordance with specifications.

The vacuum duct 32 is a duct configured to allow a charged particle beam to pass therethrough in order to avoid or reduce attenuation of the charged particle beam generated and accelerated by the accelerator 20. The charged particle beam passes through the vacuum duct 32 while being adjusted by the beam adjustment unit 31, and is guided to the superconducting magnet device 300 at a predetermined deflection angle (e.g., φ 1).

The superconducting magnet device 300 to which the charged particle beam has been guided further deflects the charged particle beam by using a magnetic field generated by a superconducting coil (for example, a superconducting coil 312A) so that the isocenter O (the affected part of patient) is irradiated with the charged particle beam at a predetermined irradiation angle (for example, θ1). The charged particle beam deflected by the superconducting magnet device 300 is emitted from an irradiation nozzle (not shown) toward the isocenter O. The irradiation nozzle is provided inside a treatment room provided with a treatment table for a patient.

The acceleration control unit 40 controls the accelerator 20 and the beam transport system 30. For example, the acceleration control unit 40 permits emission of charged particle beams, sets current values of electromagnets provided in the accelerator 20 and the beam transport system 30, and the like. The acceleration control unit 40 is communicatively connected to a control unit 330 of a superconducting magnet device 300 to be described below.

Next, the superconducting magnet device 300 will be described. The superconducting magnet device 300 includes circuits 310A to 310F, cryostats 320A and 320D, and the control unit 330.

The circuit 310A includes a power source 311A and a superconducting coil 312A. The power source 311A may have the same configuration as the power source 111A of the superconducting magnet device 100, and the superconducting coil 312A may have the same configuration as the superconducting coil 112A of the superconducting magnet device 100. The circuits 310B to 310F, the power sources 311B to 311F, and the superconducting coils 312B to 312F may have the same configurations as those of the circuit 310A, the power source 311A, and the superconducting coil 312A, respectively.

In addition, although not illustrated, the circuits 310A to 310F include a protective resistor and a breaker corresponding to the protective resistor 113A and the breaker 114A of the superconducting magnet device 100. In addition, a detector (not illustrated) corresponding to the detector 140A of the superconducting magnet device 100 is provided for each of the superconducting coils 312A to 312F.

In the present embodiment, the power source 311A included in the circuit 310A supplies a current to the power source 311A also included in the circuit 310A, but does not supply a current to the superconducting coils 312B to 312F included in the other circuits 310B to 310F. Likewise, while the power sources 311B to 311F supply currents to the superconducting coils included in the same circuit, the power sources do not supply a current to the superconducting coils included in the other circuits. That is, the superconducting coils 312A to 312F are connected to the power sources 311A to 311F neither in series nor in parallel. In other words, it can be said that the superconducting coils 312A to 312F are in a mutually independent relationship in terms of electrical circuitry.

In addition, in the present embodiment, the superconducting coils 312A to 312C are accommodated in the same cryostat 320A. That is, the superconducting coils 312A to 312C are in a relationship in which they are mutually independent in terms of electrical circuitry but are thermally connected. Furthermore, the superconducting coils 312A to 312C may be connected to each other by a heat transfer mechanism such as the heat transfer mechanism 250 of the superconducting magnet device 200. Likewise, since the superconducting coils 312D to 312F are accommodated in the same cryostat 320D, the superconducting coils are in the relationship in which they are mutually independent in terms of electrical circuitry but are thermally connected.

The control unit 330 controls operations of the superconducting magnet device 300. To be specific, the control unit 330 controls the current supplied from the power sources 311A to 311F of the circuits 310A to 310F to the superconducting coils 312A to 312F. The control unit 330 may be a control circuit including one or more processors such as a central processing unit (CPU) and a memory. These functions may be realized by a processor operating according to a program readably stored in the memory. The program may be stored in, for example, a storage device inside the superconducting magnet device 100.

Note that the control unit 330 may be configured to include hardware such as integrated circuits represented by a field programmable gate array (FPGA) or an application specific integrated circuit (ASIC) instead of or in addition to the processors and memory. The control unit 330 may be realized by a single control circuit, or may be realized by a plurality of control circuits which share the functions of the control unit 330. In addition, like the control unit 130 of the superconducting magnet device 100, the control unit 330 may include power source control units corresponding to the respective power sources and an integrated control unit that integrates the power source control units.

For example, during treatment, in order to irradiate the isocenter O with a charged particle beam at a desired irradiation angle θ, the control unit 330 sets the current from each of the power sources. Then, when the control unit 330 completes the current setting, a signal indicating the start of treatment is sent from the control unit 330 to the acceleration control unit 40. In this state, for example, when an operator presses the treatment start button (not shown), the acceleration control unit 40 receives the signal, and the acceleration control unit 40 issues a command for permitting emission of a charged particle beam to the accelerator 20 to start treatment. When the treatment is completed, a treatment end signal is sent from the control unit 330 to the acceleration control unit 40, and the acceleration control unit 40 having received the signal stops the emission of the charged particle beam. Note that sharing of the functions of the acceleration control unit 40 and the control unit 330 can be changed as appropriate. Alternatively, the functions of the acceleration control unit 40 and the control unit 330 may be realized by a single control unit, or an additional control unit in charge of some of the functions may be provided. In addition, the acceleration control unit 40 does not permit emission of a charged particle beam while the control unit 330 performs the current adjustment processing for the superconducting coils. That is, the superconducting magnet device 300 does not emit a charged particle beam while the current adjustment processing for the conductive coils is performed.

Here, a case in which quenching occurs in any one of the superconducting coils 312A to 312F will be described. For example, for the current adjustment for the superconducting coil 312B when quenching occurs in the superconducting coil 312A, the same processing as in the flowchart shown in FIG. 2, 5, 7, or 8 can be performed. In addition, whether to perform the current adjustment processing for a superconducting coil when any superconducting coil is experiencing quenching can be chosen as appropriate in accordance with the magnitude of the influence from the superconducting coil experiencing quenching. The magnitude of the influence from the superconducting coil experiencing quenching may vary depending on the positional relationship, the thermal connection relationship, and the like of the superconducting coils. Table 1 shows an example of the relationship between the superconducting coils experiencing quenching and the superconducting coils to be subjected to the current adjustment processing.

**[Table 1]**

| Superconducting coils experiencing quenching | Coils to be subjected to current adjustment processing |
|---|---|
| Superconducting coil 312A | Superconducting coil 312B |
| Superconducting coil 312B | Superconducting coil 312A |
| | Superconducting coil 312C |
| Superconducting coil 312C | Superconducting coil 312B |
| Superconducting coil 312D | Superconducting coil 312E |
| Superconducting coil 312E | Superconducting coil 312D |
| | Superconducting coil 312F |
| Superconducting coil 312F | Superconducting coil 312E |

Table 1 shows an example in which the superconducting coil adjacent to and thermally connected to the superconducting coil experiencing quenching is to be subjected to the current adjustment processing. Here, when a plurality of superconducting coils are arranged side by side in a predetermined direction, superconducting coils that are adjacent to each other are referred to as "adjacent superconducting coils". The direction in which the plurality of superconducting coils are arranged is, for example, the direction of a plane orthogonal to the direction of the magnetic field generated by the superconducting coil. Note that, other components (e.g., a support member, a wall portion of a cryostat, or the like) may be interposed between adjacent superconducting coils.

For example, when the superconducting coil 312A experiences quenching, the current adjustment processing is performed for the superconducting coil 312B adjacent to and thermally connected to the superconducting coil 312A according to the flowchart of FIG. 2, 5, 7, or 8. On the other hand, the current adjustment processing is not performed on the superconducting coil 312D that is adjacent to but is not thermally connected to the superconducting coil 312A. This is because the superconducting coil 312B receives both the influence of an overvoltage due to the electromagnetic induction from the superconducting coil 312A and the thermal influence made from the temperature rise of the superconducting coil 312A, and therefore is more likely to experience quenching than the superconducting coil 312D which would not be thermally affected. In addition, when the superconducting coil 312B or the superconducting coil 312E that is thermally connected to the superconducting coils on both sides experiences quenching, the current adjustment processing is performed on the superconducting coils on both sides. As described above, by limiting the target of the current adjustment processing to the superconducting coil adjacent to and thermally connected to the superconducting coil experiencing quenching, the current adjustment processing can be performed on the superconducting coil having a relatively high likelihood of experiencing quenching.

Next, Table 2 shows another example of the relationship between the superconducting coils experiencing quenching and the superconducting coils to be subjected to the current adjustment processing.

**[Table 2]**

| Superconducting coils experiencing quenching | Coils to be subjected to current adjustment processing |
|---|---|
| Superconducting coil 312A | Superconducting coil 312B |
| | Superconducting coil 312C |
| Superconducting coil 312B | Superconducting coil 312A |
| | Superconducting coil 312C |
| Superconducting coil 312C | Superconducting coil 312A |
| | Superconducting coil 312B |
| Superconducting coil 312D | Superconducting coil 312E |
| | Superconducting coil 312F |
| Superconducting coil 312E | Superconducting coil 312D |
| | Superconducting coil 312F |
| Superconducting coil 312F | Superconducting coil 312D |
| | Superconducting coil 312E |

Table 2 shows an example in which the superconducting coil thermally connected to the superconducting coil experiencing quenching is to be subjected to the current adjustment processing. For example, when the superconducting coil 312A experiences quenching, the current adjustment processing is performed for the superconducting coil 312C that is not adjacent to but is thermally connected to the superconducting coil 312A, in addition to the superconducting coil 312B according to the flowchart of FIG. 2, 5, 7, or 8. The temperature of the superconducting coil accommodated in the same cryostat and thermally connected to the superconducting coil experiencing quenching is likely to increase under the influence of the temperature rise of the superconducting coil experiencing quenching. Therefore, by setting the target of the current adjustment processing to the superconducting coil thermally connected to the superconducting coil experiencing quenching, it is possible to perform the current adjustment processing on the superconducting coil likely to be thermally affected by the superconducting coil experiencing quenching.

Next, Table 3 shows another example of the relationship between the superconducting coils experiencing quenching and the superconducting coils to be subjected to the current adjustment processing.

**[Table 3]**

| Superconducting coils experiencing quenching | Coils to be subjected to current adjustment processing |
|---|---|
| Superconducting coil 312A | Superconducting coil 312B |
| | Superconducting coil 312C |
| | Superconducting coil 312D |
| Superconducting coil 312B | Superconducting coil 312A |
| | Superconducting coil 312C |
| Superconducting coil 312C | Superconducting coil 312A |
| | Superconducting coil 312B |
| Superconducting coil 312D | Superconducting coil 312A |
| | Superconducting coil 312E |
| | Superconducting coil 312F |
| Superconducting coil 312E | Superconducting coil 312D |
| | Superconducting coil 312F |
| Superconducting coil 312F | Superconducting coil 312D |
| | Superconducting coil 312E |

Table 3 shows an example in which the superconducting coil adjacent to or thermally connected to the superconducting coil experiencing quenching is to be subjected to the current adjustment processing. For example, when the superconducting coil 312A experiences quenching, the current adjustment processing is performed on the superconducting coils 312B and 314D adjacent to the superconducting coil 312A and the superconducting coil 312C thermally connected to the superconducting coil 312A according to the flowchart of FIG. 2, 5, 7, or 8. Since the superconducting coil 312D is not thermally connected to the superconducting coil 312A, but is adjacent to the superconducting coil 312A, the superconducting coil 312D is easily affected by induced electromotive forces due to electromagnetic induction. Therefore, in the present example, the current adjustment processing is performed on a superconducting coil that is adjacent to but is not thermally connected to the superconducting coil experiencing quenching. As a result, the current adjustment processing can be performed on the superconducting coil that is easily affected by the superconducting coil experiencing quenching even through the coils are not thermally connected.

Note that the relationships shown in Tables 1 to 3 are examples, and the method of selecting a superconducting coil to be subjected to the current adjustment processing is not limited to the above. For example, the current adjustment processing may be performed on a superconducting coil adjacent to the superconducting coil experiencing quenching, regardless of a thermal connection.

In the superconducting magnet device 300, power sources 311A to 311F are provided for the superconducting coils 312A to 312F, respectively. During operations, a current may be simultaneously supplied to the superconducting coils 312A to 312F from the power sources 311A to 311F, but a power source that supplies a current may be selected as necessary. That is, when the isocenter O is irradiated with a charged particle beam at a predetermined irradiation angle θ, there may be no need to supply a current to all the superconducting coils 312A to 312F. Therefore, for example, a superconducting coil to which power is supplied may be selected in accordance with the irradiation angle θ to the isocenter O as shown in Table 4.

**[Table 4]**

| | Superconducting coil 312A | Superconducting coil 312B | Superconducting coil 312C | Superconducting coil 312D | Superconducting coil 312E | Superconducting coil 312F |
|---|---|---|---|---|---|---|
| I | Current supplied | - | - | - | - | - |
| II | Current supplied | Current supplied | - | - | - | - |
| III | - | Current supplied | Current supplied | - | - | - |
| IV | - | - | Current supplied | - | - | - |
| V | - | - | - | Current supplied | - | - |
| VI | - | - | - | Current supplied | Current supplied | - |
| VII | - | - | - | - | Current supplied | Current supplied |
| VIII | - | - | - | - | - | Current supplied |

| | | | | | | |
|---|---|---|---|---|---|---|
| * "-" indicates no power supplied | | | | | | |

For example, when a charged particle beam passes through only the magnetic field region generated by the superconducting coil 312A, a current is supplied only to the superconducting coil 312A (pattern I). Furthermore, for example, when a charged particle beam passes through the magnetic field region generated by the superconducting coil 312A and the magnetic field region generated by the superconducting coil 312B, a current is supplied to the superconducting coil 312A and the superconducting coil 312B (pattern II). As described above, by selecting the superconducting coil to which power is supplied in accordance with the region through which the charged particle beam passes (as a result, at the irradiation angle θ), it is possible to curb unnecessary supply of current to the superconducting coil, thereby reducing power consumption.

In addition, when the superconducting coil to which the current is supplied is appropriately selected, for example, the superconducting coil to be subjected to the current adjustment processing may be selected according to the selection state as shown in Table 5.

**[Table 5]**

| Pattern | Coils experiencing quenching | Coils to be subjected to current adjustment processing |
|---|---|---|
| II | Superconducting coil 312A | Superconducting coil 312B |
| II | Superconducting coil 312B | Superconducting coil 312A |
| III | Superconducting coil 312B | Superconducting coil 312C |
| III | Superconducting coil 312C | Superconducting coil 312B |
| VI | Superconducting coil 312D | Superconducting coil 312E |
| VI | Superconducting coil 312E | Superconducting coil 312D |
| VII | Superconducting coil 312E | Superconducting coil 312F |
| VII | Superconducting coil 312F | Superconducting coil 312E |

For example, when the superconducting coil 312B experiences quenching in the pattern II, the control unit 130 performs the current adjustment processing for the superconducting coil 312A according to the flowchart of FIG. 2, 5, 7, or 8. On the other hand, when the superconducting coil 312B experiences quenching in the pattern III, the control unit 130 performs the current adjustment processing for the superconducting coil 312C according to the flowchart of FIG. 2, 5, 7, or 8. Even when the superconducting coil 312B experiences quenching likewise as described above, the control unit 130 may determine the target of the current adjustment processing in accordance with the state of current supplied to each superconducting coil at that time.

The dimensions, materials, shapes, relative positions of components, and the like described above may be changed according to the structure of a device to which the present invention is applied or various conditions. It is not intended to be limited to the particular terms and embodiments used in the description, other equivalent components may be utilized by those skilled in the art, and other variations and modifications may be made to the embodiments without departing from the spirit or scope of the present invention. Also, features described in connection with one embodiment of the present invention may be used with other embodiments, even if not explicitly described above.

### Reference Signs List

10 Particle beam therapy system
20 Accelerator
30 Beam transport system
100 Superconducting magnet device
111A, 111B Power source
112A, 112B Superconducting coil
130 Control unit
140A, 140B Detector

## Claims

1. A superconducting magnet device comprising:
a first superconducting coil;
a second superconducting coil;
a first power source configured to supply a current to the first superconducting coil and not to supply a current to the second superconducting coil;
a second power source configured not to supply a current to the first superconducting coil but to supply a current to the second superconducting coil;
a first detector configured to detect quenching of the first superconducting coil; and
a control unit configured to perform first adjustment processing of adjusting a current to be supplied from the second power source to the second superconducting coil in response to detection of quenching of the first superconducting coil by the first detector.

2. The superconducting magnet device according to claim 1,
wherein the control unit reduces the current to be supplied to the second superconducting coil by the second power source in the first adjustment processing to be lower than a current before the first adjustment processing is performed.

3. The superconducting magnet device according to claim 1,
wherein the control unit sets the current to be supplied to the second superconducting coil by the second power source in the first adjustment processing to 0 [A].

4. The superconducting magnet device according to claim 1, further comprising:
a first breaker configured to interrupt a current supplied from the first power source to the first superconducting coil,
wherein the control unit causes the first breaker to interrupt the current supplied to the first superconducting coil when the first detector detects quenching of the first superconducting coil.

5. The superconducting magnet device according to claim 1, further comprising:
a second detector configured to detect quenching of the second superconducting coil; and
a second breaker configured to interrupt a current supplied from the second power source to the second superconducting coil,
wherein the control unit causes the second breaker to interrupt the current supplied to the second superconducting coil when the second detector detects quenching of the second superconducting coil during the first adjustment processing.

6. The superconducting magnet device according to claim 1,
wherein the control unit performs the first adjustment processing in response to quenching of the first superconducting coil detected by the first detector and a temperature of the second superconducting coil being equal to or higher than a first threshold value that is lower than a superconducting transition temperature of the second superconducting coil.

7. The superconducting magnet device according to claim 1,
wherein the control unit performs the first adjustment processing in response to quenching of the first superconducting coil detected by the first detector and a temperature gradient of the second superconducting coil being equal to or greater than a predetermined value.

8. The superconducting magnet device according to claim 1,
wherein the control unit ends the first adjustment processing when a temperature of the first superconducting coil experiencing quenching becomes equal to or lower than a second threshold value.

9. The superconducting magnet device according to claim 1,
further comprising a cryostat configured to accommodate the first superconducting coil and the second superconducting coil.

10. The superconducting magnet device according to claim 9,
further comprising a heat transfer mechanism configured to thermally connect the first superconducting coil and the second superconducting coil in the cryostat.

11. The superconducting magnet device according to claim 9, further comprising:
a third superconducting coil accommodated in the cryostat; and
a third power source configured not to supply a current to the first superconducting coil and the second superconducting coil but to supply a current to the third superconducting coil,
wherein the second superconducting coil, the first superconducting coil, and the third superconducting coil are provided side by side in this order in the cryostat, and
the control unit also performs second adjustment processing of adjusting a current to be supplied to the third superconducting coil from the third power source in response to detection of quenching of the first superconducting coil by the first detector.

12. The superconducting magnet device according to claim 9, further comprising:
a third superconducting coil accommodated in the cryostat; and
a third power source configured not to supply a current to the first superconducting coil and the second superconducting coil but to supply a current to the third superconducting coil,
wherein the first superconducting coil, the second superconducting coil, and the third superconducting coil are provided side by side in this order in the cryostat, and
the control unit also performs second adjustment processing of adjusting a current to be supplied to the third superconducting coil from the third power source in response to detection of quenching of the first superconducting coil by the first detector.

13. The superconducting magnet device according to claim 9, further comprising:
a third superconducting coil accommodated in a cryostat that is different from the cryostat in which the first superconducting coil and the second superconducting coil are accommodated; and
a third power source configured not to supply a current to the first superconducting coil and the second superconducting coil but to supply a current to the third superconducting coil,
wherein the second superconducting coil, the first superconducting coil, and the third superconducting coil are provided side by side in this order, and
the control unit also performs second adjustment processing of adjusting a current to be supplied to the third superconducting coil from the third power source in response to detection of quenching of the first superconducting coil by the first detector.

14. The superconducting magnet device according to any one of claim 11 or 13,
wherein the control unit is capable of performing a first control pattern in which the first power source and the second power source supply currents to the first superconducting coil and the second superconducting coil, respectively, and a second control pattern in which the first power source and the third power source supply currents to the first superconducting coil and the third superconducting coil, respectively,
the control unit performs the first adjustment processing when the first detector detects quenching of the first superconducting coil during the first control pattern, and
the control unit performs the second adjustment processing when the first detector detects quenching of the first superconducting coil during the second control pattern.

15. The superconducting magnet device according to any one of claims 1 to 13,
wherein the first superconducting coil and the second superconducting coil are adjacent to each other.

16. The superconducting magnet device according to any one of claims 1 to 13,
wherein the first detector detects quenching of the first superconducting coil by detecting a voltage of the first superconducting coil.

17. A particle beam therapy system comprising:
the superconducting magnet device according to any one of claims 1 to 13;
an accelerator configured to generate and emit a charged particle beam; and
a beam transport system configured to transport a charged particle beam emitted from the accelerator to the superconducting magnet device.

18. The particle beam therapy system according to claim 17,
wherein the accelerator does not emit a charged particle beam while the control unit performs current adjustment.

19. A method of controlling a superconducting magnet device including
a first superconducting coil,
a second superconducting coil,
a first power source configured to supply a current to the first superconducting coil and not to supply a current to the second superconducting coil, and
a second power source configured not to supply a current to the first superconducting coil but to supply a current to the second superconducting coil, the method comprising:
detecting quenching of the first superconducting coil; and
adjusting a current to be supplied from the second power source to the second superconducting coil in response to detection of quenching of the first superconducting coil in the detecting.
